# EUROPEAN PATENT APPLICATION

(11) **EP 1 815 785 A1**
(43) Date of publication of application: **08.08.2007**
(21) Application number: 06075235.9
(22) Date of filing: 02.02.2006
(51) Int. Cl.: A61B 5/00, G01N 33/487, C12Q 1/54

(54) **Cassette-tape formed diagnostic device for fluid diagnostic**

(71) Applicant: Bioception B.V.i.o., 2628 XH Delft (NL)
(72) Inventor: Van Halsema, Frans Emo Diderik, 3902 CN Veenendaal (NL); Arnoldussen, Eduard Antonius Joannes, 6961 PH Eerbeek (NL)
(74) Representative: Winckels, Johannes Hubertus F.

(57) **Abstract**

The invention describes a diagnostic device in a substantially fluidtight housing in which a diagnostic or test material is transported via an aperture in the housing for (multiple, continuous or discontinuous) measurements of a compound or condition of the environment, e.g. a liquid medium. Preferably the measurement is an optical or electrical measurement. Data from the measurement are transmitted wirelessly to a receiver station which is connected to a computer(network) for further processing and/or display of the data.

## Description

The invention relates to the field of diagnostics, in particular biological diagnostic devices, more in particular 'automated' diagnostic devices, i.e. devices which function without human intervention.

In recent time the field of diagnostics has undergone a radical change in that miniaturization and automation have been introduced. This has led to several high-tech applications in which miniaturised probes have been developed for measuring biological *parameters in situ, in vitro and in vivo* in the human body or in other biological systems. These applications have been equipped in many instances with transmission systems for wireless transmission of the measurements.

Examples of such diagnostic devices are abundant in the patent literature: US 2004/180391 describes a multipurpose probe for measuring physiological parameters such as glucose levels, pH, electric current, and the like, in a patient's body. WO 00/30534 describes a spherical shaped integrated circuit (IC) which can be equipped with transducers or sensors for specific measurements in biological systems. Also a wireless capsule for *in vivo* disease diagnosing has been described in US 2005/148842 and US 2005/154277. Herein the capsule is able to contain a micro-spectrometer or a micro-biosensor. Also in other fields small diagnostic devices are known, such as in culturing of cells and/or micro-organisms (WO 03/78563), for testing liquids (US 6,564,155; EP 1 228 358) and for detecting leaks of toxic compounds to the environment (WO 04/044607).

The disadvantage of many of these diagnostic devices is that they are quite expensive and/or need complicated specialised probes or sensors.

Another disadvantage is that the biological media in which the measurements are made contaminate the sensor surface (consisting of e.g. proteins, cells, biofilm), hampering proper measurement by inhibiting the active surface or introducing unknown or unpredictable sensor drift.

Thus, a need remains for an easy to manufacture, easy to operate, cheap diagnostic device which is able to perform at least one diagnostic assay, and which can operate without human intervention. Ideally such a device would be a disposable device.

### SUMMARY OF THE INVENTION

The present invention now offers a solution for this problem. In its most general embodiment it concerns a diagnostic device comprising a fluidtightly sealed housing wherein is comprised:
a) at least one spindle for accommodating a support having test or diagnostic areas;
b) an actuator for rotating said at least one spindle;
c) an aperture in the fluidtightly sealed housing for contact of at least one of said test or diagnostic areas with the environment;
d) a means for detecting a change in the material property of a said at least one of said test or diagnostic areas after or during passing said aperture;
e) a means for transmitting of detection data.

In a preferred embodiment said diagnostic device comprises at least one spindle which accommodates a set of spools (10, 15) for winding a test or diagnostic strip comprising said test or diagnostic areas. Alternatively, said diagnostic device comprises at least one spindle which accommodates a circular disc with said test or diagnostic areas. In the diagnostic the means for rotating said at least one spindle is selected from the group consisting of a torsion spring, a motor driven shaft, and a piezo motor or any magnetically driven means of providing motion.

The invention further comprises a diagnostic device according to the invention, wherein the change in the material property of a test or diagnostic area is a change in color or in light intensity. In that case, the means for detecting a change in the test/diagnostic strip comprise an optical means, preferably a combination of a light source and a colour detector, a photo diode or a spectrophotometer. An especially preferred embodiment is a device wherein the measurement takes place at the site of the aperture.

Alternatively, the change in the material property can be a change in impedance or electrical resiststance. In such a case, the means for detecting a change are an ohmmeter (resistance meter) such as a Wheatstone bridge.

An alternative preferred embodiment is a device, wherein the transmitting of data is wireless.

Another embodiment of the invention is a diagnostic test system comprising a diagnostic device according to the invention and a remote receiver, which can also be used for data processing, connection to a computer or computer network and/or display.

The diagnostic device and/or the diagnostic system of the invention can, of course, be used for performing diagnostic measurements.

### DESCRIPTION OF THE FIGURES

The accompanying drawings, which are hereby incorporated into and constitute a part of this specification, illustrate preferred embodiments of the invention and, together with the description, serve to explain the principles of the invention
Fig. 1 shows a schematic representation of a device according to the invention.
Fig. 2 shows a schematic representation of an alternative embodiment of the invention
Fig. 3 shows a schematic representation in a plane square to the plane of fig. 1 and 2, showing a further alternative embodiment of the invention.
Fig. 4 shows a schematic overview of a diagnostic system according to the invention.

### DETAILED DESCRIPTION OF THE INVENTION

The device of the invention is made to perform multiple measurements independently, which measurements are transmitted to a remote data collector for further processing or display. The measurements can be taken in an aqueous environment, such as bodily fluids, culture media, or in open water, such as lakes, seas and rivers, but the measurements can equally well be taken in oily fluids or organic solvents. It is also possible that the measurements are taken in a dry environment, if suitable test/diagnostic strips en measuring conditions are available.

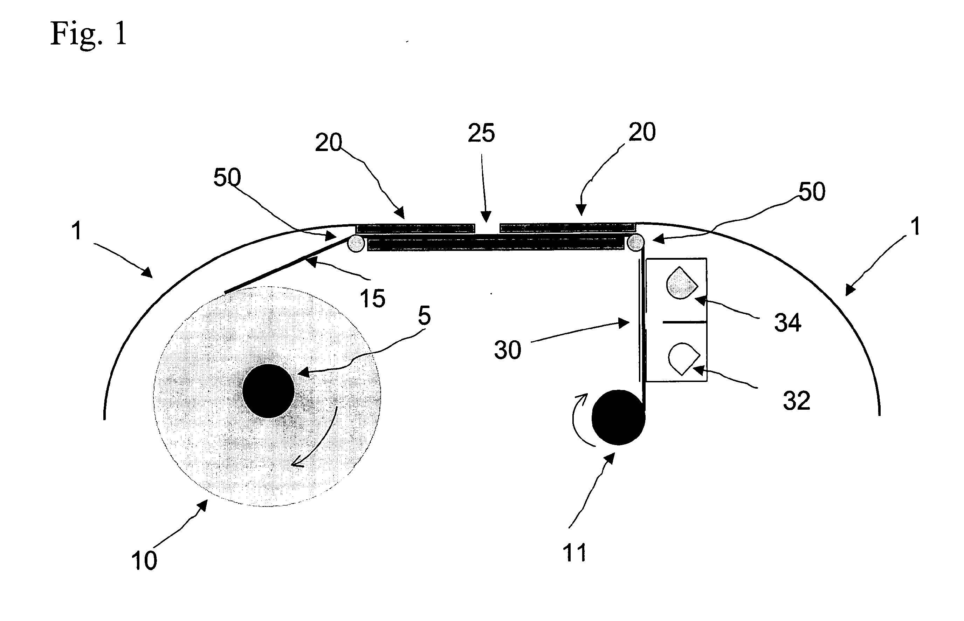

Since the device is predominantly made to measure in liquid environments, the device of the invention has a substantially fluid tightly sealed housing (see fig. 1). This housing can be of any material, but light plastic materials are preferred, such as polypropylene, polyethylene, and copolymers of these. The term 'substantially' in this respect means that most of the housing, except for the aperture where the test or diagnostic area is exposed to the environment, is fluidtight to prevent fluid leaking in and reacting with a not yet exposed test or diagnostic area or damaging the electronic parts of the device.

Inside the housing at least one spindle is provided which is used to accommodate a support which comprises more than one test or diagnostic area. The spindle or spindles are capable of rotating or being rotated around their axis, thereby transporting (rotating) the support with the test or diagnostic areas. In one embodiment inside the housing a set of spools is positioned on two spindles between which a test/diagnostic strip can be transported. The mechanism is comparable to that of cassette tapes, where a tape is (initially) wound around a first spool en by playing (i.e. rotating the spindle and with it the spool) is unwound from said first spool and wound around a second spool. In the present device, the 'tape' is formed by a test/diagnostic strip, which will be detailed below.
In a second embodiment the support is a disc which is provided with test or diagnostic areas. Also here, movement of the spindle (rotation) causes the disc to rotate.
The movement of at least one of the spindles is controlled by an actuator. In the embodiment with the spools it is preferred that both spools move synchronously together in the same winding/unwinding direction or that only one spool is activated and which, by traction of the test/diagnostic strip is able to move the second spool. As in cassette tapes, preferably rotation is accomplished by the spool which is 'empty' at the start, and which thus pulls and winds the test/diagnostic strip. By pulling the test/diagnostic strip, said strip will unwind from the initially 'full' spool, by passively rotating said spool. To this end, said spool should preferably be able to rotate freely, at least in the unwinding direction.

The actuator that provides the rotation of at least one spindle can be an axle, which is driven by a motor, for which the power is supplied by a (miniature) power supply such as a conventional battery. Several of such systems are known from the above mentioned cassette tape systems. It is also possible that the actuator is a torsion spring, which is put under tension when constructing the device and which, by releasing its tension, is able to slowly rotate the spindle, possibly connected to the spindle via a barrel with a toothed rim and a subsequent train of toothed wheels. In fact, all types of actuators, which are e.g. known from the wristwatch and clock manufacturing industry, where they are used to move the hands that indicate the time, would be useful in the present invention. Such torsion springs, which can be adjustable with respect to speed of releasing, are commercially available.

The rotation system of the spindle(s), however, is preferably rotating in discrete steps, wherefore the needs will be discussed in more detail below. Such a discrete rotation is possible e. g. in the motor driven system, by switching the motor on and off according to a predetermined schedule, as in a stepping motor. Regulatory systems (e.g. the electronic circuits which would perform this function) are commonly available. In mechanically driven systems, discrete transport is made possible by the use of toothed wheels or cam(shaft)s, e.g. as in clocks and watches. The speed of rotation depends on the type of measurements and the reaction times involved in those. The speed also depends on the lengths of the interval between discrete measurements, which lengths can vary with need for or interest in multiple measurements per time unit. The speed can also be a function of the results of the measurements, i.e. a next movement can be initiated when a previous measurement is finished successfully or has reached a threshold level.

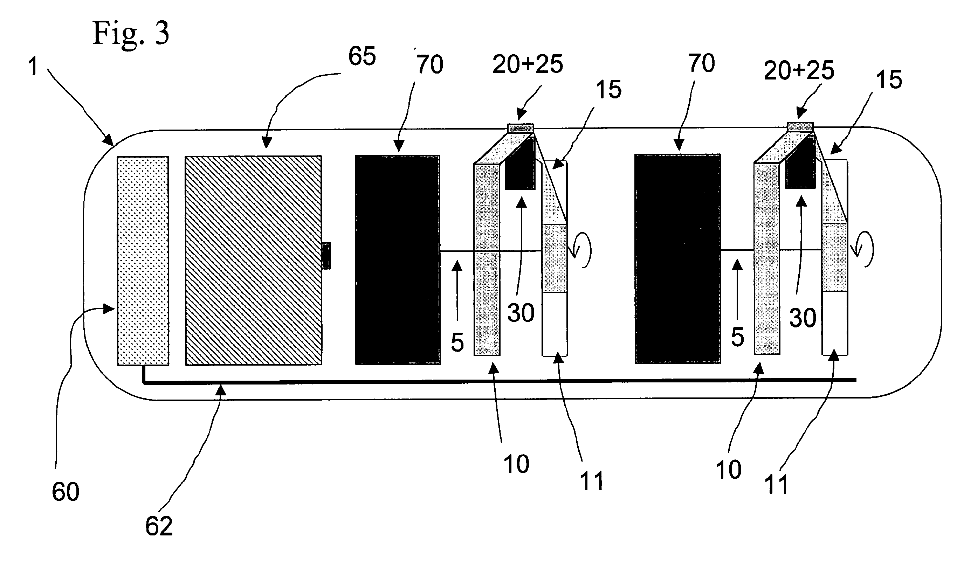

As is indicated in the Figures, the transport of the test/diagnostic strip is guided mainly through the positions of the spools and the structure that ensures exposure of the test/diagnostic strip to the environment. Also in case of a disc the rotation is dependent on the distance between the test or diagnostic areas on the strip, which needs to be exposed to the environment. Said place of exposure is an aperture in the housing, where the test/diagnostic area is able to contact the environment. To maintain the fluid tightness of the housing this should be constructed in such a way that no fluid can enter the housing at the sites where the test/diagnostic strip or disc enters or leaves the aperture. In this structure, two sheets of the same of different material are used, such as sheets of glass, polystyrene, polypropylene, nylon, aramid, or any other suitable material of which the sheet which faces the outside of the device has an aperture, and of which the sheet which faces the inside of the device doe not have an aperture. The sheets are attached to the inner side of the housing in such a way that the aperture of the outside facing sheet coincides or falls within the opening of the housing and that both sheets cover at least the whole opening in the housing. The connection of the sheets with the housing is made fluid tight by sealing the sheets to the housing, e.g by gluing or by thermosetting (i.e. if the sheets and the housing are made of thermoplastic material, they can be tightly sealed by heating them and allowing them to cool down). Also the sheets are connected fluid tightly (according to similar techniques of gluing or melting together), but space is left for the strip or disc to enter and be transported between the sheets. For a strip this is preferably established by having a slot in the bottom sheet, which guides the strip (see Fig. 3). A fluid tight seal can also be established by using fluid magnet material (magnetite coated with a surfactant and dispersed in a viscous, oily material such as oleic acid) introduced in a slit between two sheets of permanet magnetic material through which the test strip is transported.

The test/diagnostic strip normally is a conventional diagnostic test/diagnostic strip, such as is used, for instance, in dipstick assays. The test/diagnostic strip may be any solid support to which an analyte binding agent and/or detectable agent (indicator) can be attached. It can thus be made of any material which is able to accommodate the reagents that are used for the diagnostic reaction and which is able of being transported between the spools. Such materials can for instance be paper, poly ethylene teraphtalate, nitrocellulose, plastic, polyethylene or any other suitable material. Care should be taken that the absorbent, capillary action of the strip material and the analyte are not too big, otherwise, in prolonged contact of one piece of the test/diagnostic strip or disc with the aqueous environment, fluids will be transported through the paper and reach and contaminate the part of the test/diagnostic strip or disc which is not yet exposed. Alternatively, this can be solved by inserting non absorbent pieces of material at discrete sites in the test/diagnostic strip or disc to stop the capillary movement of the fluid. Of course these discrete sites should be spaced at a distance such that they are flanking the exposed part of the test/diagnostic strip or disc, when the test/diagnostic strip or disc is transported into its new position. A further advantage of these discrete non-absorbent areas can be that they can be used for control/zero-line measurements.

Preferably the test/diagnostic strip or disc at least for the areas at which the test/diagnosis takes place is transparent, i.e. being translucent in such a way that light which is passed through the test/diagnostic strip or disc is sufficient for an optical measurement of the diagnostic reaction. Thus, the test/diagnostic strip or disc may be totally translucent, or opaque, or any variation in between.

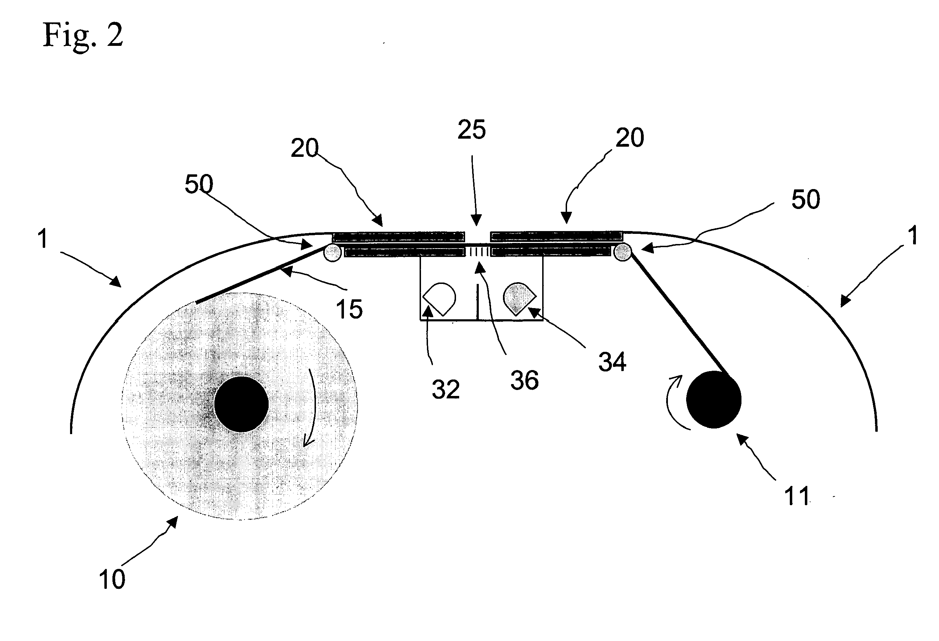

The measurement may be any type of measurement which is known to be performed using a diagnostic area, e.g. measurement of pH, temperature, (dissolved) oxygen, metabolites, enzymes, proteins, nucleic acids, inorganic compounds, etc. Test/diagnostic strips for measuring these compounds or environmental conditions (parameters) are known in the art, and often are commercially available. Preferably the reaction results in a reaction which is detectable optically, i.e. a change of color or translucency of the test/diagnostic strip or disc. In order to detect such a color or translucency change, light should be shone on or through the test area. This can be done in two ways: either the light is the light from a light source outside the housing which enters the diagnostic device through the aperture, where the test/diagnostic strip or disc is exposed to the environment (see Fig. 2) or the light is provided by a light source contained within the device. In both cases the light source can be e.g. a laser light source or a LED (see Fig. 1). Said laser light source LED can be chosen to emit only a very limited range of wavelengths, i.e. a specific colour or colour range.

The test/diagnostic strips or discs may be obtained commercially or may be prepared for the specific purpose of the diagnostic measurements. Specific preparation can include immobilizing diagnostic reagents on the solid state, such as specific binding partners or binding components, with which is meant any molecule or compound that is configured to specifically bind to or react with an analyte of interest. For example, and without limitation, the analyte binding partner may comprise antigens, antibodies, receptors, other polypeptides, peptides, haptens, lectins, nucleic acid, including oligonucleotides, or small molecules, such as indicators or dyes, or combinations or chemical complexes of the above. In one embodiment the analyte binding agent is an antigen that is specific for an antibody that is to be detected in a sample. In another embodiment the analyte binding compound is an antibody that is specific for an antigen of interest. The test or diagnostic area may contain additional layers for filtering the analytes from the environment. For optical detection mostly one of the assay components is labelled e.g. with a fluorescent dye, or detection is accomplished through the use of so-called indicators, which change color as a result from e.g. an enzymatic reaction. Also envisaged are the use of dyes, which can be pH or chemically sensitive, in such a way that the colour changes with a different pH or concentration of a specific chemical compound. Thus, the diagnostic reaction may be any of the known diagnostic reactions which can be conveniently done on a test or diagnostic area, such as enzyme immunoassays (EIA), enzyme linked immunoassays (ELISA), and the like.

Detection can be an optical detection of a change in colour or light intensity and said detection can either be performed by lighting the test or diagnostic area through the aperture of by lighting the test or diagnostic area with a laser or LED light source. This optical detection is performed by any apparatus that can be used to measure light intensity, wavelength or color, such as a spectrophotometer, a photo diode, a photometer, a spectrofluorimeter or a colorimeter. Preferably the latter is used in the form of a colour (RGB)-sensor that is commercially available. All the above-mentioned optical measuring instruments are available commercially, also in miniature format.

The detection can also use a change in impedance or electrical resistance. This can e.g be caused by using or changing the electrical charges of the components (proteins, peptides, DNA, polymers, etc.) of the analytes, the compounds that are detected or the strip itself. The detection will then be performed by measuring the resistance or impedance using e.g. an Ohmmeter, such as a Wheatstone bridge. This technology has been known in the field and is e.g. applied in biosensors.

The optical or electrical measuring instruments will provide an output signal to a data collecting and transmission means. Said data collecting and transmission means normally can comprise an A/D converter, an amplifier for amplifying the optical detection signals and a radio frequency or ultrasonic transmitter. It is envisaged that for each measurement one value is transmitted or that the measurement and transmission is continuous, e.g. for analysing the kinetics of the reaction at the diagnostic test or diagnostic area. It is also possible that the measurement is continuous, but that transmission takes only place with intervals, thereby transmitting the measurement value(s) at the time of transmission. Depending on the electronic and logical circuitry one or more values per time unit are transmitted.

These transmissions are received at a centralized receiver unit, which preferably is connected to a computing and/or displaying unit, such as a stand-alone computer or internet-connected computer, or a graphic screen, with or without memory capabilities, or a polygraph. Basically any data acquisition and display system can be coupled to the central receiver station.

Data transmission between the diagnostic device and the central receiver station is preferably performed with commercially available open radio frequency wireless interface protocols (such as BlueTooth or Zigby) or with a similar custom wireless interface protocol. Wireless communication can equally well be established by means of ultrasonic transmission and reception. Communication between the central receiver station and the Internet or any other computer or server system will preferably comply with open standard communication protocols such as TCP/IP.

The test or diagnostic strip or disc can be divided in subsections. One embodiment in which a subsection is used is where a control reaction ideally needs to take place to obtain useful measurement data. In such a case it is envisaged that the test or diagnostic area on said strip or disc has two sections (basically two halves), one for the specific reaction, i.e. one half containing the specific diagnostic reagents, and one for the control reaction, i.e. the other half is used to standardize the measurement. Such standard controlled measurements are very useful to subtract any aspecific reactions from the obtained specific reaction signal, or to compensate for inadvertent shifts in either the quality of the test or diagnostic strip or disc and/or the quality of the optical measurement and transmission circuitry.

It is also possible to engineer more than one diagnostic reaction on a test or diagnostic strip or disc, e.g. by dividing the strip or disc in multiple compartments (areas), each with specific diagnostic reagents, possibly each with their own control compartment. In such a way multiple parameters can be measured simultaneously. It is also possible to stack more than one diagnostic disc or strip, or rather spools with diagnostic strips on top of each other. Preferably such a stack of spools is driven by the same actuator, to provide synchronous movement of the two or more test or diagnostic strips. Each strip then is led to its own second spool via its own aperture. Alternatively, in the case of discs, multiple discs are moved by their own actuators, of which the movements should be aligned to achieve proper synchronisation. Also here each disc will have its own aperture. Since in such an embodiment each test or diagnostic strip or disc reacts at its own aperture, also each test or diagnostic strip or disc should have its own optical detection system. Alternatively, a system which can be used for multiple measurements, e.g. through a movable optical sensor, can be used.

The diagnostic device of the present invention can be used in any system where continuous or frequent diagnostic data are desired. The major advantages of the diagnostic device are that it is fluid-tight, which means that it can be employed also in aqueous environments or in the free air; its small size, which enables employment in or near living systems; the fact that, due to the low cost of the components and assembly, it is disposable, which means that no elaborate retrieval (and cleaning) is necessary; its independence with relation to the sort of test that can be performed; which means that any known test which can be performed on a test or diagnostic area would be applicable for the current system; and its wireless characteristic, which means that the diagnostic measurements can be performed remote from the (receiving and) display system, e.g. in closed containers or in the (human) body.

For applications *in vivo*, the housing of the device can be coated so as to minimise any immunological or graft-vs-host ractions.

Parameters which can be measured include, but are not limited to, acidity (pH); (dissolved) gasses, such as oxygen or carbon dioxide, etc.; fluids, such as water (rain), alcohol (in brewing), and oils; cell metabolites, such as ethanol, lactic acid, urea, carbohydrates (sugar levels in fluids), etc.; inorganic ionic species, such as ammonia, nitrate, chloride, phosphate, etc.; organic molecules, such as proteins, hormones, herbicides, insecticides, etc.; and immunological compounds, such as antibodies, antigens, T cells, B cells, macrophages, etc. Accordingly, the diagnostic device of the present invention can be employed in various environments, such as in or near the human or animal body, in fermentation equipment, e.g. in beer brewing or microbiological production, in industrial process monitoring, e.g. production of chemicals, food or feed, in ecological systems, such as waste water treatment and environmental protection.

Specific uses are envisaged for the control of fermentation processes, in which one or more of the diagnostic devices of the invention are added to the medium for measurement of pH, dissolved oxygen, dissolved carbon dioxide, ethanol and/or other desired parameters. A continuous or frequent measurement of the value of these parameters ensures feedback to the operator, which allows the operator to adjust settings of the process, or to adjust addition/removal of ingredients for optimal production. The device can be used in traditional fermentor systems or in disposable fermentor bags.

In another embodiment the diagnostic device is used to measure data *in vivo.* This can be accomplished by introducing the device into the body of an animal or human through surgery or injection. It is also possible to attach the diagnostic device to a venapunction or infusion system, where at certain intervals, blood can be drawn from the subject to be evaluated by the diagnostic device.

Many more applications of the diagnostic device will be apparent to the person skilled in the art. These are all included in the scope of the present invention.

The invention will now be illustrated by the following examples, which are not meant to limit the invention in any way.

### EXAMPLES

Figure 1 shows a typical embodiment of the invention, wherein a teststrip (15) is wound around a spool (10), sitting on a spindle (5) and the strip is transported via strip guiders (50) first along an aperture (25) in the housing (1) and then to a detection means (30), which comprises a light source (32) and a light/color sensor (34). After being transported through the detection means, the strip is guided to a second spool (11) on a second spindle, where it is wound. At the site of the aperture (25) the strip is guided through a seal (20) which forms part of and/or is closely aligned to the housing (1). An alternative embodiment is shown in Figure 2, where only the position of the detection means (30) has changed. As can be seen the seal (20) now contains a transparent slit (36) at the site of the aperture (25) to allow light from the light source (32) to reach the test strip (15) at the site of the aperture (25). In the embodiment depicted in Figure 2 only a part of the seal at the aperture is made transparent, but alternatively, of course, the whole seal can be of transparent material.
A further alternative embodiment of the invention is shown in Figure 3. This figure, which shows a section of the device at a plane square to the plane of view in Figures 1 and 2, two parallel teststrips according to Figure 2 (i.e. where the measurement takes place at the site of the aperture) are present. In this view each set of spools (not shown) is driven by an actuator, e.g. and axle (5) powered by a an electromotor, piezomotor or torsion spring (70). In Figure 3, the axle is connected with the second spindle, i.e. the spindle where the used strip is wound. Transport of the strip (15) is caused by this spindle, thus guiding the strip along the seal and aperture (20+25), where measurement takes place via the light source and the sensor present in the sensor housing (30). Power for the actuator is optionally given by a battery (65). Electrical signals from the measurement are guided to a printed circuit board (60) where signal handling takes place. The circuit board (60) bears an antenna (62) for wireless transmission of the data. Although the circuit board and battery are pictured in Fig. 3 at the left side of the device, both the circuit board, the battery and the antenna can be placed at any side of the device, subject to space constraints or other manufacturing reasons. The circuitry which connects the circuit board with the sensor in the sensor housing (30) is not shown.

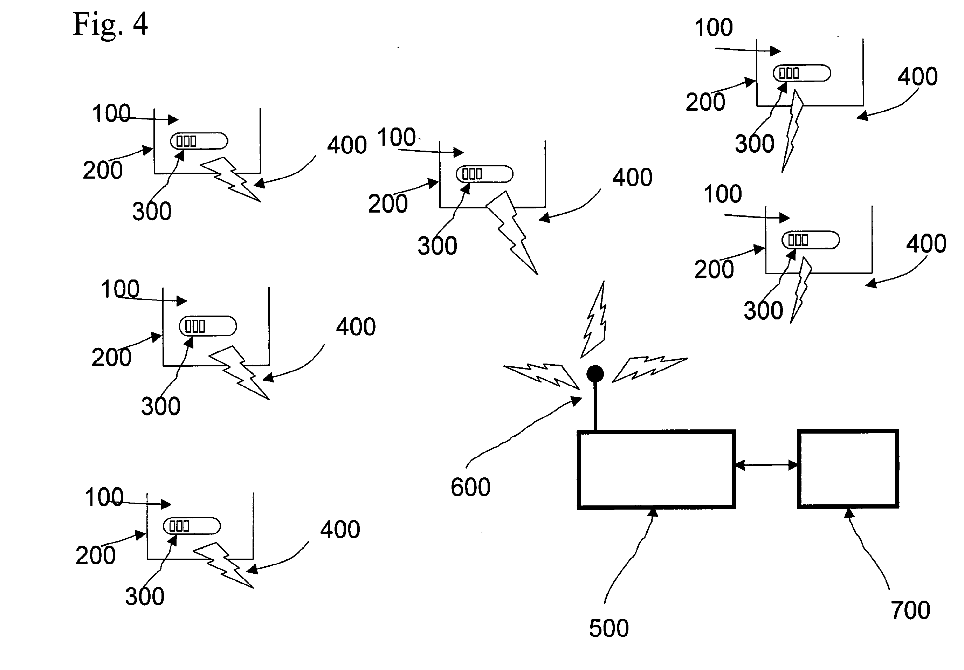

Figure 4 shows an overview of a diagnostic system according to the invention. Several diagnostic devices (300) are placed in a medium (100), e.g. a liquid medium, in a container or environment (200). The data from the measurement are transmitted wirelessly (400), picked up by an antenna (600) connected to a transceiver station (500). Said transceiver station is capable of bidirectional communication with each of the diagnostic devices/sensor systems. The transceiver (500) further communicates with a computer systems, network or internet (700), where the data is further processed and/or displayed.

## Claims

1. A diagnostic device comprising a substantially fluidtightly sealed housing (1) wherein is comprised:
a) at least one spindle (25) for accommodating a support having test or diagnostic areas;
b) an actuator for rotating said at least one spindle;
c) an aperture (5) in the fluidtightly sealed housing (1) for contact of at least one of said test or diagnostic areas (20) with the environment;
d) a means for detecting a change in the material property of a said at least one of said test or diagnostic areas after or during passing said aperture;
e) a means for transmitting of detection data.

2. A diagnostic device according to claim 1, wherein said at least one spindle accommodates a set of spools (10, 15) for winding a test or diagnostic strip comprising said test or diagnostic areas.

3. A diagnostic device according to claim 1, wherein said at least one spindle accommodates a circular disc with said test or diagnostic areas.

4. A diagnostic device according to any of claims 1-3, wherein the means for rotating said at least one spindle is selected from the group consisting of a torsion spring, a motor driven shaft, and a piezo motor or any magnetically driven means of providing motion.

5. A diagnostic device according to any of claims 1-4, wherein the change in the material property of a test or diagnostic area is a change in color or in light intensity.

6. A diagnostic device according to claim 5, wherein the means for detecting a change in the test or diagnostic area comprise an optical means, preferably a combination of a light source and a color detector or a spectrophotometer.

7. A diagnostic device according to claim 6, wherein the light source is the light entering through the aperture (5) in the fluidtightly sealed housing (1).

8. A diagnostic device according to any of claims 1-4, wherein the change in the material property of a test or diagnostic area is a change in impedance or electrical resistance.

9. A diagnostic device according to claim 8, wherein the means for detecting a change in the test or diagnostic area comprise an electrical means, preferably an electrical circuit, more preferably an Ohmmeter such as a Wheatstone bridge.

10. A diagnostic device according to any of claim 1-9 wherein the transmitting of data is wireless.

11. A diagnostic system comprising a diagnostic device according to claim 10 and a remote receiver for processing/displaying data or connection to a computer(network).

12. Use of a diagnostic device according to any of claims 1-10 or a diagnostic system according to claim 11 for diagnostic measurements.
